# EUROPEAN PATENT SPECIFICATION

(11) **EP 1 515 736 B1**
(45) Date of publication and mention of the grant of the patent: **26.11.2008**
(21) Application number: 03740342.5
(22) Date of filing: 26.06.2003
(51) Int. Cl.: A61K 36/28, A23L 1/03, A61P 29/00, A61P 35/00

(54) **COMPOSITIONS AGAINST INFLAMMATORY PROCESSES**
ZUSAMMENSETZUNGEN GEGEN INFLAMMATORISCHE PROZESSE
COMPOSITIONS CONTRE DES PROCESSUS INFLAMMATOIRES

(30) Priority: 26.06.2002 US 391739 P
(43) Date of publication of application: 23.03.2005
(60) Divisional application: 08010165.2
(73) Proprietor: Nestec S.A., 1800 Vevey (CH)
(72) Inventor: MALNOE, Armand, CH-1066 Epalinges (CH); CAVIN, Christophe, CH-1041 Poliez-Pittet (CH); OFFORD, Cavin, Elizabeth, CH-1041 Poliez-Pittet (CH); GRIGOROV, Martin, CH-1066 Epalinges (CH)
(74) Representative: Lock, Graham James
(86) International application number: PCT/EP2003/006718
(87) International publication number: WO 2004/002505

(56) References cited:
- JP-A- 11 318 387
- US-A- 5 905 089
- US-A- 5 952 033
- US-B1- 6 391 375
- DATABASE WPI Section Ch, Week 200008 Derwent Publications Ltd., London, GB; Class B04, AN 2000-090491 XP002257244 & JP 11 318387 A (KANEBO LTD), 24 November 1999 (1999-11-24)
- DATABASE BIOSIS [Online] BIOSCIENCES INFORMATION SERVICE, PHILADELPHIA, PA, US; October 2000 (2000-10) CASTRO VICTOR ET AL: "Inhibition of the transcription factor NF-kappaB by sesquiterpene lactones from Podachaenium eminens" Database accession no. PREV200100067197 XP002257242 & PLANTA MEDICA, vol. 66, no. 7, October 2000 (2000-10), pages 591-595, ISSN: 0032-0943
- CASTRO V ET AL: "Study of sesquiterpene lactones from Milleria quinqueflora on their anti-inflammatory activity using the transcription factor NF-kappaB as molecular target" PHYTOCHEMISTRY, PERGAMON PRESS, GB, vol. 53, no. 2, January 2000 (2000-01), pages 257-263, XP004291284 ISSN: 0031-9422
- DATABASE BIOSIS [Online] BIOSCIENCES INFORMATION SERVICE, PHILADELPHIA, PA, US; August 1999 (1999-08) DE LA PUERTA R ET AL: "Inhibition of leukocyte eicosanoid generation and radical scavenging activity by gnaphalin, a lipophilic flavonol isolated from Helichrysum picardii" Database accession no. PREV200000198755 XP002257243 & PLANTA MEDICA, vol. 65, no. 6, August 1999 (1999-08), pages 507-511, ISSN: 0032-0943
- PAMBUCCIAN G. ET AL.: REVISTA DE CHIRURGIE ONCOLOGIE RADIOLOGIE ORL OPHTHALMOLOGIESTOMATOLOGIE SERIA STOMATOLOGIE, vol. 28, no. 1, 1981, pages 61-68,
- PATEL V K ET AL.: "Cichorum Intibus Linn" THERAPIE, DOIN, PARIS, FR, vol. 38, no. 4, 1983, pages 405-414, XP001062501
- T. A. VAN BEEK ET AL.: "Bitter sesquiterpene lactones from Chicory Roots" J. AGRIC. FOOD CHEM., vol. 38, no. 4, 1990, pages 1035-1038,
- HARSH PAL BAIS AND GA RAVISHANKAR: "Cichorum intybus L -" J. SCI. FOOD. AGRIC., vol. 81, 2001, pages 467-484,

## Description

The present invention generally relates to compositions. More specifically, the present invention relates to compositions that include thermally processed extruded extracts of chicory, to enhance health in humans and animals. The chicory contains a desirable and effective quantities of sesquiterpene lactones, including α-methylene-γ-butyrolactone (α-MGBL) upon heating

### Background of the invention

The need to enhance health in mammals has involved and continues to involve on-going research efforts and discoveries to prevent and/or treat disease. For example, irritation or discomfort can result from inflammation in a mammal due to, for example, skin inflammation, eye inflammation, gut inflammation or the like. Further, it is generally believed that chronic inflammation may increase the risk to develop other disease or ailment, such as osteoarthritis, autoimmune disease, cancer or the like. In this regard, it is generally understood that inflammation is triggered by an enhanced transcription activity of transcription factor NF-κB that leads to the expression of proinflammatory and inflammatory enzymes and receptors. More specifically, inflammation can occur due to increased enzyme activity in the mammal, such as an increased activity of cyclooxygenase including cyclooxygenase-1, cyclooxygenase-2 or the like.

In general, food, dietary or other nutritional sources are known to contain a number of constituents or agents that are believed to be capable of protecting against disease in humans and animals. For example, oligosaccharides, such as inulin and various fructo-oligosaccharides, are reported to have prebiotic effects, such as promoting the growth of bifido- and lacto-bacteria in the gastro-intestinal tract at the expense of pathogens including, for example, *Clostridium perfringens*. See, for example, Gibson et al., Food Microbiology, 11 (6), pp. 491-498 (1994). Although most reported experimentation has been carried out in vitro, there have been reports that these oligosacchrides have a similar effect in the gut of rats and humans, In this regard, it is generally known that the promotion of growth of bifido- and lacto-bacteria through the use of oligosacchrides can provide a variety of different beneficial effects on animals and humans, such as the prevention and/or treatment of diarrhea, increased growth, improved ability to breed or other like beneficial effects which enhance health.

Inulin or other dietary agents that are believed to promote health in humans and animals as discussed above, in general, can be derived from plants or other natural sources. For example, inulin is generally known to be purified from plants that contain high concentrations of inulin, such as chicory, Jerusalem artichoke, leek and asparagus. In this regard, the plant is typically purified or otherwise treated prior to use in order to enhance a plant's flavor, such as to eliminate, or at least minimize, a bitter flavor typically associated with chicory. See, for example, U.S 4,865,852.

In general, the purified plant product is prepared by hydrolyzation with acids or enzymes. The hydrolysate is then collected and condensed to obtain the bioactive agent, such as inulin. For example, JP 63-309147 discloses grinding chicory tubers, partially hydrolyzing them with acids, and then drying the hydrolysate with or without neutralization. However, the purification of, for example, fructo-oligosaccharides and inulin, can greatly add to the cost of the dietary product. Consequently, the use of such dietary products has been generally limited to specialty food or dietary products in humans and animals.

A need, therefore, exists for a composition that includes natural ingredients, such as thermally processed chicory and/or extracts thereof, that are palatable to humans and animals, that can be inexpensively produced, and that can enhance health in humans and animals, such as the prevention and/or treatment of inflammation.

### Summary of the invention

The present invention relates to compositions which are defined in the appended claims that can be utilized to prevent and/or a treat inflammation. The compositions of the present invention include sesquiterpene lactones which include α-methylene-γ-butyrolactone (α-MGBL). processed e.g extruded chicory.

Applicants have demonstrated that chicory root extracts contain one or more phytochemicals with the ability to inhibit enzyme and/or transription activity in mammals, such as enzyme activity relating to cyclooxygenase and transcription activity relating to NF-κB. Applicants further demonstrated that thermally processed extruded chicory root extracts possess an enhanced enzyme inhibition activity relating to cyclooxygenase and/or enhanced inhibition related to NF-κB. Applicants demonstrated a relationship between the enhancement of inhibition to the generation during thermal processing of an active molecular species, namely α-methylene-γ-butyrolactone (α-MGBL).

In this regard, the inhibition of such enzyme/transcription activity was proven to prevent and/or treat inflammation in mammals. By inhibiting the inflammatory process, the risk of incidence of other disease or ailments, such as cancer, which are believed to result from inflammation (e.g., chronic inflammation) may be reduced.

To this end, in an embodiment of the present invention, a nutritional composition is provided as defined in the appended claims. Preferably, the chicory material comprises an amount of at least 0.5% by weight.

The plant material, in an embodiment, is derived from chicory

The compositions can include other dietary agents derived from the plant source in addition to the phytochemical agents. These dietary agents can include any suitable constituent capable of inhibiting enzymatic activity associated with cyclooxygenase. In an embodiment, the dietary agents in addition to phytochemicals include antioxidants, glucosamine, chondroitin sulphate, omega-3 fatty acids, any suitable other dietary agents and combinations thereof.

The present invention also provides a pet food product. The pet food product includes a starch matrix; and an effective amount of a thermally processed extruded e.g chicory extract that inludes sesquiterpene lactones which include α-MGBL capable of inhibiting enzyme activity in a mammal to reduce a risk of inflammation.

In another embodiment of the present invention, a process for preparing a nutritional food product capable of educing a risk of incidence of inflammation in a mammal is provided. The process is defined in the appended claims The nutritional food product includes at least 1 % by weight of chicory extract.

Preferably, the chicory extract is processed by defatting the chicory material to form a first chicory extract and subsequently processing the first plant extract with ethyl acetate via acid hydrolysis to form the plant extract.

The present invention also provides the use of a therapeutically effective amount of a thermally processed extruded chicory material according to the invention , for the preparation of a composition intended for reducing a risk of inflammation in a mammal at risk of inflammation. With decreased enzyme activity, the risk of inflammation in the mammal can be reduced thereby reducing a risk of other ailments or disease that may result from the inflammation, such as osteoarthritis, autoimmune disease, cancer or the like.

An advantage of the present invention is to provide an improved composition that can be utilized to reduce a risk of incidence of inflammation in mammals. In this regard, the composition is capable of inhibiting enzyme activity, the result of which is believed to treat and/or prevent inflammation.

Another advantage of the present invention is to provide an improved composition that includes chicory material containing one or more phytochemicals capable of inhibiting an enzymatic activity, such as relating to cyclooxygenase, which is believed to prevent and/or treat inflammation in mammals.

Yet another advantage of the present invention is to provide methods of producing improved compositions containing chicory extract that can enhance the palatability of the composition while maintaining the enzymatic inhibition properties of the plant material which are capable of reducing a risk of inflammation in mammals.

Yet still another advantage of the present invention is to provide methods of treatment and/or prevention against inflammation in mammals that include the administration of an improved composition.

Additional features and advantages of the present invention are described in, and will be apparent from, the following detailed description of the invention.

### Brief description of the drawings

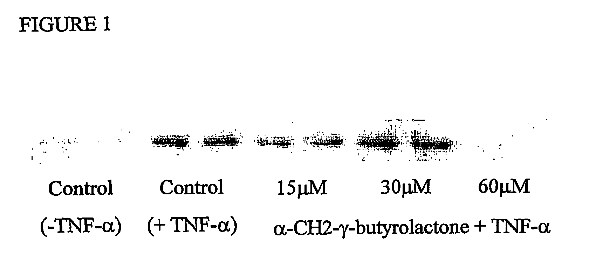

**Figure 1** illustrates the inhibition of COX-2 expression in HT29 cells by α-methylene-γ-butyrolactone (α-MGBL) pursuant to an embodiment of the present invention.

### Detailed description of the invention

The present invention relates to compositions as defined in the appended claims

Applicants have surprisingly discovered that upon thermal processing certain plants and/or plant extracts thereof, such as chicory, phytochemicals and the like, can be generated with enhanced inhibition of cyclooxygenase enzyme activity and/or enhanced transcription activity of NF-κB in mammals which is believed to reduce the risk of inflammation including, for example, skin inflammation, eye inflammation, gut inflammation, colon inflammation and the like. In an embodiment, the enzymatic activity is derived from cyclooxygenase, such as cyclooxygenase-1 and/or cyclooxygenase-2.

Applicants have demonstrated through testing that thermally processed, such as extruded, plant extracts, particularly chicory extracts, can inhibit cyclooxygenase activity as well as transcription activity of NF-κB. In particular, it is believed that the thermally processed extruded chicory extracts have a more pronounced effect on the inhibition of cyclooxygenase-2 as compared to cyclooxygenase-1. Thus, the inhibition of this type of enzyme activity is believed to reduce inflammation in mammals. By reducing inflammation, it is believed that the risk of incidence of other disease or ailments which are believed to result from inflammation, particularly chronic levels of inflammation, may be reduced. The other types of disease can include, for example, cancer, autoimmune disease, osteoarthritis, and combinations thereof.

In addition to phytochemicals as discussed above, plants, such as chicory, are known to also contain prebiotic fibers, such as oligosacchrides including inulin, that are believed to reduce cancer incidence, particularly in the colon. In this regard, Applicants believe that enhanced benefits with respect to cancer prevention and/or treatment in humans and animals can be realized due to the combined effect of prebiotic fibers and phytochemicals that can inhibit enzyme activity to prevent and/or treat inflammation in mammals, particularly chronic inflammation which may cause cancer, such as colon cancer, if the inflammation is untreated.

Applicants have also demonstrated that the enzyme inhibiting properties of the thermally processed extruded composition of the present invention are essentially unaffected by the processing conditions under which the compositions are prepared pursuant to present invention. For example, purification of the plant material made pursuant to the present invention which can be utilized to reduce the bitterness of the plant extract and thus enhance human and animal palatability has negligible, if any, effect on the nutritional properties of the resultant purified product. In this regard, resultant extract product can result from essentially crude plant extracts, such as chicory. This can eliminate the need for expensive purification or other like treatment of the plant material necessary to produce the resultant bioactive fractions.

As used herein, the term "bioactive agent" or other like terms, such as "bioactive fractions", means any constituent or constituents that can display biological activity, chemical activity or like activity in a mammal(s) that are capable of enhancing health in a mammal. Examples of bioactive agents include, for example, prebiotic fibers, phytochemicals or the like.

As used herein, the term "prebiotic" or other like terms including "prebiotic fiber" means a substance or a constituent that can promote the growth of microorganisms in mammals.

As used herein, the term "phytochemical" or other like terms including "phytochemicals" and "phytochemical agent" means any chemical produced by a plant that is believed to impart health benefits to humans and/or animals, such as the prevention and/or treatment of inflammation or other like disease.

As used herein, the term "enzyme activity" or other like terms, such as "enzymatic activity" means any suitable enzyme which can act as a catalyst during any suitable biological, chemical or other like process which is believed to effect the health in a mammal. For example, the inhibition of enzyme activity relating to cyclooxygenase is believed to reduce the risk of incidence of inflammation.

The composition can include any suitable and compatible types and amounts of constituents such that the composition can be effectively utilized to prevent and/or treat inflammation. In an embodiment, the composition includes a chicory extract that contains one or more prebiotic fibers and phytochemical agents which is capable of inhibiting enzyme activity, such as enzyme activity relating to cyclooxygenase. This is believed to be responsible for the treatment and/or prevention of inflammation.

It should be appreciated that the chicory can be processed to form an extract in a variety of different and suitable ways. In general, the chicory root, is ground, powderized or provided in any suitable form. The plant material can then be further processed in a number of different stages to produce the product extract. In an embodiment, a defatting procedure is performed on the plant material to produce an extract that results from fats removed from the plant material. The defatting procedure can be conducted under any suitable defatting process conditions with any suitable types and amounts of solvents including, for example, hexane.

In an embodiment, the resultant extract of the defatting procedure can be further processed via acid hydrolysis to produce another type of plant extract that can be added to the composition of the present invention. The acid hydrolysis procedure can be conducted under any suitable process condition with any suitable types and amounts of solvents, including, for example, ethyl acetate.

In an embodiment, the extract from the defatting procedure can be further processed via a solvent extraction procedure. The solvent extraction can be carried out under any suitable process conditions and in the presence of any suitable amount and type of solvent. In an embodiment, the solvent includes a solution of methanol ("MeOH") and water mixed in a 1:1 volume ratio. The resultant solution of the solvent extraction procedure can be further processed by evaporation of the solvent under suitable conditions to produce another extract. Alternatively, the resultant solution can be treated with an adsorbant agent, such as polyvinylpolypyrrolidone or the like, to trap polyphenols. The adsorbant agent treatment can be carried out under any suitable process conditions. Specific examples of preparing plant extracts in accordance with an embodiment of the present invention are detailed below.

In an embodiment, the ground plant raw material is processed thermally. Applicants have demonstrated that in this way the raw plant material that naturally contains sesquiterpene lactones (SQLs) is enriched in a highly active COX2 inhibitory species, namely α-methylene-γ-butyrolactone (α-MGBL). Indeed, this molecule exhibits an enhanced thermal stability compared to other SQLs. For example, this molecular species was demonstrated to inhibit inflammatory activity in part through direct interaction with transcription factor NF-κB and the inhibition of its binding to DNA. The inhibition was proposed to result from the alkylation a precise amino acid Cys³⁸ in the p65 domain of NF-κB. The inhibition results in the decrease of expression of several proinflammatory and inflammatory receptors and enzymes, among which cyclooxygenase-II. SQLs, in general, and α-MGBL in particular inhibit COX-2 actitivity and/or or its expression. Applicants have demonstrated that a-CH₂-γ-butyrolactone inhibits specifically and with high potency COX-2 activity.

In an embodiment, the prebiotic fiber(s) and phytochemical agent(s) of the composition can be derived from chicory. As previously discussed, Applicants believe that the combined effect of the prebiotic fiber and phytochemical source of chicory, can result in an enhanced chemoprotective effect such that inflammation in mammals can be treated and/or prevented. The prebiotic fiber can include any suitable amount and type including, for example, oligosacchrides, such as inulin and various fructo-oligosacchrides, soyoligosacchrides and combinations thereof.

The phytochemical agents can include any suitable type and amount such that it is capable of inhibiting COX-II enzyme activity or NF-κB transcription activity that are believed to be responsible for the prevention and/or treatment of inflammation. In an embodiment, the phytochemical agent of the plant material is capable of inhibiting cyclooxygenase activity, such as cyclooxygenase-1 and/or cyclooxygenase-2 and/or NF-κB transcription activity. By inhibiting enzyme and/or transcription activity, the phytochemicals of the present invention are believed to be capable of preventing and/or treating inflammation, including, for example, chronic inflammation. In this regard, the risk of incidence of other disease or ailments which are believed to be caused by inflammation, such as cancer, osteoarthritis, autoimmune disease, may be reduced.

In addition to the phytochemicals, the plant source can include other dietary agents which are capable of inhibiting enzyme activity. In an embodiment, the dietary agent include antioxidants, glucosamine, chondroitin sulphate, omega-3 fatty acids, the like and combinations thereof.

It should be appreciated that the composition of the present invention can include a variety of different and suitable forms. In an embodiment, the composition can include a nutritional supplement, a food preparation for humans and/or animals, pet food and/or pharmaceutical and/or functional food composition or the like. The composition can be added to the food product in any suitable amount. In an embodiment, the food product includes the plant material of the composition in an amount of at least 0.5% by weight, preferably from about 1% to about 30% by weight, more preferably about 1% to about 2% by weight.

In another embodiment, the pharmaceutical compositions containing the active ingredient α-methylene-γ-butyrolactone, fragments or extracts containing it may be in any form suitable for oral use, such as e.g. tablets, troches, lozenges, aqueous or oily suspensions, dispersible powders or granules, emulsions, hard or soft capsules, or syrups or elixirs. Compositions intended for oral use may be prepared according to any method known in the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient(s) in admixture with non-toxic pharmaceutically acceptable excipients, such as inert diluents, granulating, disintegrating and lubricating agents, which are suitable for the manufacture of tablets. The tablets may be uncoated or they may be coated by known techniques to delay disintegration and absorption in the gastrointestinal tract and thereby provide a sustained action over a longer period. Formulations for oral use may also be presented as hard gelatin capsules wherein the active ingredient is mixed with an inert solid diluent, or as soft gelatin capsules wherein the active ingredients is mixed with water or an oil medium. Aqueous suspensions contain the active material in admixture with excipients suitable for the manufacture of aqueous suspensions, such as e.g. suspending agents, dispersing or wetting agents, preservatives, coloring agents, flavoring agents, and sweetening agents. Dispersible powders and granules suitable for preparation of an aqueous suspension by the addition of water provide the active ingredient(s) in admixture with a dispersing or wetting agent, suspending agent and one or more preservatives. Additional excipients, for example sweetening, flavoring and coloring agents, may also be present.

In an embodiment, the present invention provides a pet food product that includes a starch matrix and an effective amount of a composition as defined in the appended claims
- The pet food product of the present invention can include any suitable number, type and amount of constituents and be processed in any suitable way to form a desirable product form.

In an embodiment, the present invention includes a gelatinized product which contains an amount of chicory extract. The chicory extract at least includes a source of prebiotic fibers and phytochemicals capable of inhibiting enzymatic activity in mammals which is believed to enhance health in the mammal.

In an embodiment, the chicory extract includes inulin, sufficient to provide at least about 0.25% by weight inulin, on a dry matter basis. In an embodiment, the plant materials include at least 50% by weight of inulin. For ease of handling, the plant material is preferably in a dried and comminuted or powder form. As described below, the processes utilize dried, comminuted chicory and/or extracts thereof However, it is to be understood that any suitable chicory extract may be used in any suitable form and added to the cereal product in any suitable amount.

As described below, the remaining ingredients included in the gelatinized cereal product may be any suitable ingredients commonly used in gelatinized cereal products. Usually these ingredients include a starch source and a protein source. Suitable starch sources are, for example, grains such as com, rice, wheat, beets, barley, oats, soy, and mixtures thereof. Suitable protein sources may be selected from any suitable animal or vegetable protein source. Examples include meat meal, bone meal, fish meal, soy protein concentrates, milk proteins, gluten, and the like. The choice of the starch and protein sources will be largely type of cereal product produced or other like considerations. Various other ingredients, for example, sugar, salt, spices, seasonings, vitamins, minerals, flavoring agents, fats and the like may also be incorporated into the gelatinized cereal product as desired.

The gelatinized cereal product may be produced in many different ways as desired. However, for a dried cereal product, an especially suitable way of producing the product is extrusion cooking. This may be done as is well known in the art. For example, in one suitable process, a feed mixture is fed into a preconditioner. The feed mixture is primarily made up of a starch source, a protein source, and the plant material, chicory. In an embodiment, the chicory includes at least about 1% by weight of the feed material, preferably at least about 2% by weight. In an embodiment, the amount of plant material in the food material ranges from about 10% to about 20% by weight, preferably about 10% by weight.

In the preconditioner, water or steam, or both, is mixed into the feed mixture. A sufficient amount of water or steam is mixed into the feed mixture to moisten the feed mixture. If desired, the temperature of the feed mixture may be raised in the preconditioner to about 60°C to about 90°C by weight. A suitable preconditioner is described in US 4,752,139. It should be appreciated that the use a preconditioner is not required.

The moistened feed leaving the preconditioner is then fed into an extruder. The extruder may be any suitable single or twin screw and cooking extruder. Suitable extruders may be obtained from Wenger Manufacturing Inc., Clextral SA, Bühler AG, and the like. During passage through the extruder, the moistened feed passes through a cooking zone, in which it is subjected to mechanical shear and is heated. In an embodiment, the moistened feed is heated up to a maximum temperature of up to about 150°C, and a forming zone. The gauge pressure in the forming zone is about 300 kPa to about 10 MPa as desired. If desired, water or steam, or both, may be introduced into the cooking zone. During passage through the extruder, the starch source of the moistened feed is gelatinized to provide a gelatinized matrix structure primarily of starch, protein and the plant material, chicory.

The gelatinized matrix leaving the extruder is forced through a suitable die for example, a die as described in EP 0665051. A shaped extrudate, which has a cross-sectional shape corresponding to that of the orifice of the die, leaves the die. Depending upon the conditions in the extruder and the starch source used, the shaped extrudate expands to a greater or lesser extent. The shaped extrudate is then cut into pieces using blades. The individual pieces are then dried and, if desired, coated with protective or flavoring agents, or both. After cooling, the pieces may be packed into suitable packages. Alternatively, the individual pieces may be formed into flakes and then dried.

Depending upon the ingredients used, the gelatinized cereal product may be in the form of dried kibbles suitable for use as pet foods, expanded pieces suitable for use in breakfast cereals, flakes suitable for use in breakfast cereals, and the like.

It is also possible to produce a dried cereal product by mixing together water and the ingredients of cereal product, for example, by mixing in a preconditioner. The wet mixture may then be shaped into a desired shape by using, for example, shaping rollers. The shaped mixture may then be baked in an oven, at any suitable temperature. In an embodiment, the temperature ranges from about 220°C to about 280°C for a suitable baking time. In an embodiment, the baking time ranges from about 10 minutes to about 1 hour. The dried cereal product has the appearance of a baked biscuit.

If it is desired to produce a simulated meat product which may be used in canned pet foods, any suitable process can be used. For example, the processes can include those described in US 4,781,939 and 5,132,137. In these processes, a protein source, especially a meat material, is emulsified. The meat material may be any suitable source of animal protein including for example, the muscular or skeletal meat of mammals, poultry, and fish or meat by-products such as hearts, liver, kidneys, tongue and the like, or meat meals. Vegetable protein sources may also be included if desired. The exact composition may be selected according to cost and the desired flavor. The emulsification may be carried out in any suitable equipment.

The dried chicory is added to the emulsion. Also, if desired or needed, additional protein may be added to the emulsion. The additional protein may be any protein source as mentioned above. The exact choice will depend upon availability, cost and palatability. The additional protein can be added in any suitable amount. In an embodiment, the additional protein can be added in an amount ranging from about 5% to about 35% by weight. If desired or required, fats may also be added to the emulsion. Usually the amount of fat in the emulsion must be controlled to facilitate processing and to obtain an acceptable product. However, the meat material may well contain the desired amount of fats and hence adjustment may not be necessary. Typically, at this stage the emulsion contains a maximum fat level of about 25% by weight. In an embodiment, the amount of fat in the emulsion is in the range of about 5% to 15% by weight, more preferably about 7% to about 12% by weight. The mass ratio of protein to fat in the emulsion is preferably about 1:1 to about 7:1. If added, the fats may be any suitable animal fats, such as tallow, or may be vegetable fats.

Additional ingredients such as sugars, salts, spices, seasonings, flavoring agents, minerals, and the like may also be added to the emulsion. In an embodiment, the amount of additional ingredients used ranges from about 1% to about 5% by weight of the gelatinized cereal product.

Water may also be added to provide from about 45% to 80% by weight moisture in the emulsion. If sufficient moisture is present in the meat material, water need not be added.

Once mixed, the emulsion is preferably fed through a vacuum stuffer, or similar de-aeration apparatus, to de-aerate the emulsion. This removes air which may otherwise cause disruption of the formulated emulsion product and reduce its meat-like appearance. The emulsion is then fed to an emulsion mill which subjects the emulsion to rapid mechanical heating and shearing. Any suitable emulsion mill may be used including, for example, the emulsion mill disclosed in U.S 5,132,137. Other suitable emulsion mills are commercially available under the trade name of TRIGONAL and may be obtained from Siefer Machinenfabrik GmbH & Co KG. Bahnhofstrasse 114, Postfach 101008., Velbert 1, Germany.

The temperature of the emulsion can be raised to the desired coagulation temperature in the emulsion mill in a few seconds. In an embodiment, the coagulation temperature ranges from about 100°C to about 120°C. In an embodiment, the temperature ranges from about 45°C to about 75°C as described in U.S 5,132,137. In general, the mechanical energy generated in the emulsion mill will be sufficient to heat the emulsion to the desired temperature but this may be supplemented by the injection of superheated steam.

The heated emulsion leaving the emulsion mill can be transferred to a holding tube. In the holding tube, the heated emulsion coagulates while moving slowly along the holding tube. The residence time of the heated emulsion in the holding tube is sufficient for the emulsion to have coagulated into a firm emulsion product upon reaching the exit of the holding tube. The firm emulsion product leaving the holding tube is then transferred to a cutter where it is cut into pieces, such as chunks, of size suitable for use in a pet food. The chunks have the appearance and texture of meat. The chunks may be subjected to flaking if desired. The chunks may also be formulated into a chunk-in-gravy type of product. Other procedures for producing chunks are known and may be used, such as extruding a feed mixture, cooking the feed mixture in a steam oven, and the cutting of the cooked extrudate into chunks.

If it is desired to produce a canned pet food in the form of a meat loaf, a meat batter may be prepared by emulsifying a suitable meat material to produce a meat emulsion. The meat material may be any suitable meat source, for example, as described above. Suitable gelling agents including gums, such as kappacarrageenan, locust bean gum, guar gum, xanthan gum or the like, may be added to the meat emulsion. In an embodiment, no more than about 2% by weight of gum is used. The dried chicory plant material is then added to the meat emulsion.

Additional ingredients such as sugars, salts, spices, seasonings, flavoring agents, minerals, and the like may also be added to the meat emulsion. The amount of additional ingredients used is preferably such that they make up about 0.25% to about 5% by weight of the meat batter. Water may also be added the meat emulsion to provide from about 70% to about 85% by weight. If sufficient moisture is present in the meat material, water need not be added.

The meat emulsion is then heated to a temperature above about 65°C in a mixer-cooker. Steam may be injected into the meat batter if desired. The heated meat emulsion is then again emulsified to provide a loaf batter and the loaf batter maintained at a temperature above about 60°C until filling into cans.

It should be appreciated that the gelatinized cereal product may be produced by any suitable process and not only those described above. Other types of oligosaccharides may also be included in the gelatinized cereal product such as fructo oligosaccharide and soy oligosaccharide. The soy oligosaccharides may be added in the form of soy meal or other suitable soy source.

The cereal products may be in any suitable form including; for example, dried, semi-wet and wet. However, the matrix that makes up the cereal product must be gelatinized in order to remove or destroy the sesquiterpene compounds that may be present in the plant material. It should be appreciated that the cereal product of the present invention can be made for human and/or animal consumption.

By way of example, and not limitation, examples of pet food products made pursuant to an embodiment of the present invention are illustrated below.

### EXAMPLE 1: Dried Pet Food

A feed mixture is made up of about 58% by weight of corn, about 6% by weight of corn gluten, about 23% by weight of meat and meal, dried chicory and salts, vitamins and minerals making up the remainder. The dried chicory is in the form of a chicory extract made pursuant to an embodiment of the present invention and added in an amount of about 5% or less. The feed mixture is fed into a preconditioner and moistened. The moistened feed is then fed into an extruder-cooker and gelatinized. The gelatinized matrix as it leaves the extruder is forced through a die and extruded, thus forming an extrudate. The extrudate is cut into pieces suitable for feeding to dogs, dried at about 110°C for about 20 minutes, and cooled to form pellets. It should be appreciated that part or a totality of the fat mix, or of the fat and oils used, can be added at a later stage, for example, as a coating.

The added chicory can enhance the pet's health by, for example, preventing and/or treating inflammation as previously discussed.

### EXAMPLE 2: Dried Pet Food

A dry pet food is prepared like the dried pet food of Example 1. It further includes an additional ingredient typically associated with enhancing the palatability of the dry pet food suited to cats. The added chicory can enhance the pet's health by, for example, preventing and/or treating inflammation as previously discussed.

### EXAMPLE 3: Dry Cat Food

A feed mixture is made up of about 58% by weight of com, about 6% by weight of corn gluten, about 23% by weight of chicken meal, dried chicory and salts, vitamins and minerals making up the remainder. The chicory is added in an amount of about 5% or less. As previously discussed, the added chicory can inhibit enzymatic activity which is believed to enhance health in the animal by, for example, treating and/or preventing inflammation.

The feed mixture is fed into a preconditioner and moistened. The moistened feed is then fed into an extruder-cooker and gelatinized. The gelatinized matrix as it leaves the extruder is forced through a die and extruded, thus forming an extrudate. The extrudate is cut into pieces suitable for feeding to cats, dried at about 110°C for about 20 minutes, and cooled to form pellets. At this stage, a lyophilized powder of one or more strains of the *Lactobacillus* species, such as *Lactobacillus rhamnosus* NCC2583 (CNCM I-2449), *Lactobacillus acidophilus* NCC2628 (CNCM I-2453) and *Enterococcus faecium* SF68 (NCIMB 10415), is applied to the pellets. A sufficient amount of the powder is applied to the pellets such that the corresponding dietary intake amount for the cat is from about 1.0E+07 to about 1.0E+9 cfu / day. In this regard, a portion of the powder is mixed into a first mass of pellets which are subsequently bagged. A second portion of the powder is measured and mixed with a lipid carrier which is then sprayed on to a second mass of pellets. The pellets are bagged after the coating has dried sufficiently at 50-60°C for some minutes.

### EXAMPLE 4: Canned Pet Food and Supplement

A mixture is prepared from about 73% of poultry carcass, pig lungs and beef liver (ground), about 16% of wheat flour, about 2% of dyes, vitamins, and inorganic salts. This mixture is emulsified at 12°C and extruded into the form of a pudding. Dried chicory in the form of an extract made pursuant to an embodiment of the present invention is added to the emulsion in an amount of about 5% or less. The emulsion is then cooked at a temperature of 90°C. It is cooled to 30°C and then cut into chunks. About 45% of the chunks are mixed with about 55% of a sauce that is prepared from about 98% of water, about 1% of dye and about 1% of guar gum. Tinplate cans are filled with the chunk and sauce mixture and sterilized at 125°C for about 40 minutes.

As a probiotic supplement to be mixed with the pet food before serving, additional packaging in sachet form with strains of the following *Lactobacillus* species are provided *Lactobacillus rhamnosus* NCC2583 (CNCM I-2449), *Lactobacillus acidophilus* NCC2628 (CNCM I-2453) or *Enterococcus faecium* SF68 (NCIMB 10415). The corresponding dietary intake of the supplement for the pet is from about 106-10¹² cfu/day, depending on the type ofpet, e.g., a cat or a dog, and on the pet's physical factors, such as body mass. The supplement is packaged such that it is removably attached to the can, together with feeding directions.

By way of example, and not limitation, experimental testing as described in detail below was conducted to demonstrate the effectiveness of the present invention.

### In Vitro Testing 1

The inventors have conducted a number of experimental tests to demonstrate the beneficial effects of the present invention. In general, human colon cancer cell cultures were prepared and treated with varying amounts of chicory extracts over a period of about 48 hours to evaluate the effects of chicory with respect to cyclooxygenase activity. The preparation and experimental testing procedures and results are discussed below in greater detail:

### Preparation of Cell Culture

Colon cancer cell line HT-29 was obtained from the American Type Culture Collection and cultured in McCoy's 5A medium supplemented with 10% fetal bovine serum (FBS), 25mg/ml gentamicin. HT-29 cells were treated with a number of different chicory extracts made pursuant to an embodiment of the present invention as detailed below.

### Preparation of Chicory Extracts

Four different chicory extracts, namely Extracts A-D, were prepared pursuant to an embodiment of the present invention. Initially, a 40 gram (g) and a 10g sample of chicory ground to powder form were each sieved at 0.5mm. The samples were then processed to remove fats by mixing the samples with hexane for about thirty minutes at room temperature. 600 milliliters (ml) of hexane was added to the 40g chicory sieved sample, and 150ml was added to the 10g sieved sample. The hexane was evaporated under vacuum at about 50°C to form Extract A.
Extract B was prepared by first defatting 40g of ground chicory powder as previously discussed. The defatted sample was hydrolyzed in 300ml of an acid, such as HCl, in a boiling water bath for about 20 minutes. After cooling and centrifugation (8000 rpm, 5 minutes, 10°C), the solution was extracted with 150ml of a solvent, such as ethyl acetate, which is commercially available, such as from MERCK. The solvent is evaporated to dryness. After further drying on anhydrous sodium sulfate under vacuum at about 50°C, Extract B was formed.
Extracts C and D were prepared as follows. First, a 10g sample of ground chicory powder was defatted as previously discussed. The second part of the defatted powder is extracted with about 250ml of a solvent/water mix, such as a 1:1 volume ratio of MeOH and water in solution. The extraction is performed under stirring at room temperature (e.g., about 20°C to about 25°C) for about 30 minutes. After centrifugation, the solution is divided into two equal volumes. To the first volume part of the solution, the organic solvent is evaporated under vacuum at about 50°C. The remaining aqueous phase is freeze dried to form Extract C.
The second volume part of the solution is treated with about 2g of polyvinylpolypyrrolidone under stirring for about 30 minutes to trap polyphenols. The adsorbant material is removed by filtration, centrifugation or the like. The organic solvent is evaporated under vacuum at about 50°C. The remaining aqueous phase is freeze dried to form Extract D.

### Measurement of PGE2 production

The effects of various chicory extracts, namely, Extracts A-C, on the biosynthesis of PGE2 were analysed in a human colon cell line HT-29. The biosythesis of PGE2 provides an indication of the level of enzyme activity such as cyclooxygenase, as it is generally known that cyclooxygenase can act as a catalyst to produce. PGE2 from, for example, Arachidonic acid. Thus, the effects of chicory extract on the enzyme activity of cyclooxygenase can be evaluated based on this experiment as described below.
Cells were grown for 48 hours in their media supplemented with 0.1% Bovine Serum Albumin (BSA) and 10 µM Arachidonic Acid. Chicory extracts were then added to these media at concentrations of 50, 100 and 200µg/ml for either 21 hours or for 15 hours. Another set of cell samples was prepared by adding chicory extract B to the HT-29 cells as discussed above followed by a co-incubation in the presence of 10ng/ml of Tumor Necrosis Factor alpha (TNF) for 6 hours. It is generally believed that the addition of TNF will stimulate inflammation.

The quantity of PGE2 in cell media was then determined with the PGE2 Monoclonal Enzyme Immunoassay Kit (Cayman Chemical) according to the manufacturer's instructions. Briefly, 25 or 50 µl of the medium, along with the serial diluted PGE2 standard samples, was mixed with appropriate amount of acetylcholineesterase-labeled tracer and PGE2 anti-serum and incubated at room temperature for 18 hours. After the wells were emptied and rinsed with wash buffer, 200 µl of Ellman's reagent that contained substrate for acetylcholine esterase was added. The enzyme reaction was carried out in a slow shaker at room temperature for 1 hour. Results were measured using a microplate reader at 415 nm and normalized to micrograms of protein.
The test results indicated that the chicory extract B, which was made with ethyl acetate as previously discussed, had the most pronounced effect on the inhibition of cyclooxygenase activity as evidenced by a decrease in the amount of PGE2 measured. In general, the decrease in cyclooxygenase activity was more pronounced with increasing amounts of chicory extract.
Further, the test results indicated that the chicory extract B had a more pronounced effect on the inhibition of cyclooxygenase activity in the HT-29 cell line with TNF as compared to the HT-29 cell line without TNF. This suggests that the inhibition of cyclooxygenase activity may be more influenced by a specific inhibition of cyclooxygenase-2 as compared to cyclooxygenase-1. The results are presented in Table 1

**Table 1. Effect of chicory extracts with ethylacetate on PGE-2 synthesis in HT-29 cells**

| Addition | PGE-2 levels (% of control) - TNF alpha | PGE-2 levels (% of control) + TNF-alpha* |
|---|---|---|
| Control (vehicle alone) | 100 | 100 |
| Chicory Extract B (100 mcg/ml) | 95 | 80 |
| Chicory extract B (200 mcg/ml) | 33 | 0.7 |

| | | |
|---|---|---|
| * Stimulation of enzyme activity by TNF-alpha was 10-fold | | |

### In Vitro Testing 2

### 1. Inhibitory effects of α-CH₂-γ-butyrolactone on PGE2 synthesis

The inventors have conducted a similar experimental test to demonstrate the antiinflammatory effects of alpha-methylen-gamma-butyrolactone. In general, inhibition of PGE2 synthesis by alpha-methylen-gamma-butyrolactone has been showed in HT29 cells stimulated by TNF-alpha. To this end, cells were cultured and passed in Mc Coy's 5A medium supplemented with arachidonic acid (10 microliter) and bovine serum albumin (0.01%). Cells were treated by alpha-methylen-gamma-butyrolactone (dissolved in methanol) at the following doses: 15, 30, 60 microM or by solvent alone during 21 h including or not stimulation with TNF-alpha (10 ng/ml) for 6 h. PGE2 analysis was performed using an Elisa test on a medium sample.

**Table 2: PGE₂ concentration in pg/ml of medium in HT 29 cells treated with α-CH₂-γ-butyrolactone with and without TNF-α.**

| | **pg PGE₂/ml of medium - TNF-α** | **% of control** | **pg PGE₂/ml of medium + TNF-α** | **% of control** |
|---|---|---|---|---|
| Méthanol control | 2594 | **100** | 18941 | **730** |
| α-CH₂-γ-butyrolactone 15 µM | 2814 | **108** | 13286 | **512** |
| α-CH₂-γ-butyrolactone 30 µM | 1970 | **76** | 9349 | **360** |
| α-CH₂-γ-butyrolactone 60 µM | 1144 | **14** | 1310 | **50** |

### 2. Inhibitory effects of α-CH₂-γ-butyrolactone on COX-2 expression

Western blot analysis: cells lysate were prepared by treating HT 29 cells (treated as above for PGE-2 determinations, *i.e.* in presence or absence of TNF-alpha and with or without α-CH₂-γ-butyrolactone) with lysis buffer (150 mM NaCl, 10mM Tris HCl pH 8, 1% Tween 20, EDTA pH 8 1mM, proteases inhibitors, PEFA, (Merck), DETC (Aldrich)). Lysates were sonicated for 5 s, and centrifugated at 10000g for 5 min. Proteins concentration were estimated by the Bradford method which was standardized using bovine serum albumine.

Whole cell extracts (25 µg) were boiled in Laemmli sample buffer and resolved by SDS-PAGE on 10% gel using the discontinuous polyacrylamide gel system. Non specific binding sites were blocked by incubating the membrane in 5% dried milk phosphate buffered saline solution containing 0.1% of tween-20 overnight at 4°C.
Blots were hybridized in a 1:1000 dilution of a goat polyclonal anti COX-2 and also COX-1 antibody (C-20, Santa Cruz Biotechnology) in 5% milk PBS for 1h at room temperature followed by 1h of washing in 1% milk PBS.
Subsequently, blots were probed with a secondary donkey anti-goat antibody horseradish peroxidase conjugated. After washing for 1h, the complex was detected using ECL Western blotting reagents (Amersham)
The results are shown in Figure 1.

### 3. Specific inhibition of COX-2 activity by α-CH₂-γ-butyrolactone

The Stressgen StressXpress™ cyclooxygenases activity Kit provides a method to measure specifically cyclooxygenases COX-1 and COX-2 in a variety of biological fluids. The kit uses a specific chemiluminescent substrate (aromatic hydrocarbon molecule) to detect the peroxidative activity of COX enzymes. After inhibition by specific compounds (α-CH₂-γ-butyrolactone and NS- 938, a specific COX-2 inhibitor from Cayman Chemical Company, both dissolved in methanol) the direct residual activity of cyclooxygenase is measured by addition of the chemiluminescent substrate and arachidoinc acid (50 µM). Light emission begins immediately and chemiluminescent signal (Relative Luminescence Units - RLU- is measured after 30 minutes (luminometer Tecan). The amount of light emitted is directly proportional to the COX activity.

In this test, α-CH₂-γ-butyrolactone was found to inhibit specifically and with high potency COX-2 activity as did the reference coumpound NS-398. The results are presented in Table 3.

**Table 3: Measurement of cyclooxygenases activities (COX-1, COX-2)**

| | COX-1 activity (RLU) | % of control | COX-2 activity (RLU) | % of control |
|---|---|---|---|---|
| **Vehicle alone (methanol)** | 4115 | 100 | 26450 | 100 |
| **α-CH₂-γ-butyrolactone (40 µM)** | 4807 | 117 | 12823 | 49 |
| **NS-398 (1mM)** | 4063 | 99 | 1794 | 7 |

## Claims

1. A composition comprising a thermally processed extruded chicory extract which comprises sesquiterpene lactones which include α-methylene-γ-butyrolactone (α-MGBL), wherein the chicory extract is obtained by defatting chicory plant material to form a first plant extract and subsequently processing the first plant using extract acid hydrolysis and solvent extract with ethyl acetate to form the chicory extract.

2. A composition according to claim 1 including an effective amount of one or more phytochemical agents which include a sesquiterpene lactone having as an active fragment α-methylene-γ-butyrolactone.

3. A composition according to any one of claims 1 to 2, in which the composition comprises 0.5% extruded chicory extract by weight.

4. A composition according to any one of the preceding claims, which further comprises a dietary agent selected from the group consisting of antioxidants, glucosamine, chondroitin sulphate, omega-3 fatty acids and combinations thereof.

5. A food product which is a pet food comprising:
a starch matrix; and
a composition according to any one of claims 1 to 4.

6. A food product according to claim 5, selected from the group consisting of a nutritional supplement, a nutritionally complete food product, a food preparation, a cereal product, a pet food and a pharmaceutical and/or functional food preparation.

7. A process of preparing a nutritional food product for preventing or treating inflammation in a mammal, the process comprising the steps of:
providing an extruded chicory extract obtained by defatting chicory plant material to form a first, plant extract and subsequently processing the first plant extract using acid hydrolysis and solvent extraction with ethyl acetate to form the chicory extract and processing the chicory extract and one or more food ingredients to form a nutritional food product that includes at least 0.5% by weight of the chicory extract.

8. A process according to claim 7, in which the chicory extract includes one or more phytochemical agents capable of inhibiting the enzyme activity of cyclooxygenase and/or the NF-kB transcriptional factor.

9. A process according to claim 7 or 8, in which the extruded chicory extract further includes a dietary agent selected from the group consisting of antioxidants, glucosamine, omega-3 fatty acids or a combination thereof.

10. A composition according to any of claims 1 to 4 for use in therapy.

11. Use of a composition according to any of claims 1 to 4 in the manufacture of a medicament for use in the treatment of inflammation or cancer, or reducing the risk of inflammation, osteoarthritis, an autoimmune disease or cancer in a mammal.

12. Use according to claim 11, in which the medicament comprises 0.5% by weight of a composition according to any one of claims 1 to 4.

## Patentansprüche

1. Zusammensetzung umfassend einen thermisch behandelten extrudierten Chicoreeextrakt, der Sesquiterpen-Laktone umfasst, die α-Methylen-γ-butyrolakton (α-MGBL) beinhalten, worin der Chicoreeextrakt durch Entfetten von Pflanzenmaterial des Chicoree erhalten wird, um einen ersten Pflanzenextrakt auszubilden und anschließend den ersten Pflanzenextrakt unter Verwendung von einer sauren Hydrolyse und einer Lösungsmittelextraktion mit Ethylacetat zu bearbeiten, um den Chicoreeextrakt auszubilden.

2. Zusammensetzung nach Anspruch 1, die eine wirksame Menge von einem oder mehreren phytochemischen Mitteln umfasst, die ein Sesquiterpen-Lakton beinhalten, das als ein aktives Fragment α-Methylen-γ-butyrolakton aufweist.

3. Zusammensetzung nach einem der Ansprüche 1 bis 2, in denen die Zusammensetzung 0,5-Gew.-% von extrudiertem Chicoreeextrakt umfasst.

4. Zusammensetzung nach einem der vorhergehenden Ansprüche, die weiterhin ein Nahrungsergänzungsmittel ausgewählt aus der Gruppe bestehend aus Antioxidantien, Glucosamin, Chondroitinsulfat, Omega-3-Fettsäuren und Kombinationen davon umfasst.

5. Lebensmittelprodukt, das eine Tiernahrung darstellt, umfassend;:
eine Stärkematrix, und
eine Zusammensetzung nach einem der Ansprüche 1 bis 4.

6. Lebensmittelprodukt nach Anspruch 5, ausgewählt aus der Gruppe bestehend aus einem Nahrungsergänzungsmittel, einem ernährungsphysiologisch vollständigen Lebensmittelprodukt, einer Lebensmittelpräparation, einem Getreideprodukt, einer Tiernahrung und einer pharmazeutischen und/oder einer funktionellen Lebensmittelpräparation.

7. Verfahren zum Herstellen eines ernährungsphysiologischen Lebensmittelproduktes zur Verhinderung oder Behandlung einer Entzündung in einem Säugetier, wobei das Verfahren die Schritte umfasst:
Bereitstellen eines extrudierten Chicoreeextraktes, der durch Entfetten von Pflanzenmaterial des Chicoree erhalten wurde, um einen ersten Pflanzenextrakt zu bilden und anschließend den ersten Pflanzenextrakt unter Verwendung einer sauren Hydrolyse und einer Lösungsmittelextraktion mit Ethylacetat zu bearbeiten, um den Chicoreeextrakt zu bilden und
Bearbeiten des Chicoreeextraktes und eines oder mehrerer Lebensmittelbestandteile, um ein ernährungsphysiologisches Lebensmittelprodukt zu bilden, das mindestens 0,5 Gew.-% des Chicoreeextraktes beinhaltet.

8. Verfahren nach Anspruch 7, wobei der Chicoreeextrakt ein oder mehrere phytochemische Mittel beinhaltet, die die Enzymaktivität von Cyclooxygenase und/oder den NF-κB Transkriptionsfaktor hemmen können.

9. Verfahren nach Anspruch 7 oder 8, wobei der extrudierte Chicoreeextrakt weiterhin ein Nahrungsergänzungsmittel umfasst, ausgewählt aus der Gruppe bestehend aus Antioxidantien, Glucosamin, Omega-3-Fettsäuren oder einer Kombination davon.

10. Zusammensetzung nach einem der Ansprüche 1 bis 4 zur Verwendung in einer Therapie.

11. Verwendung einer Zusammensetzung nach einem der Ansprüche 1 bis 4 bei der Herstellung eines Medikaments zur Verwendung bei der Behandlung einer Entzündung oder einer Verringerung des Risikos einer Entzündung, einer Osteoarthritis, einer Autoimmunerkrankung oder Krebs in einem Säugetier.

12. Verwendung nach Anspruch 11, wobei das Medikament 0,5 Gew.-% einer Zusammensetzung nach einem der Ansprüche 1 bis 4 umfasst.

## Revendications

1. Composition comprenant un extrait de chicorée extrudé ayant subi un traitement thermique, qui comprend des lactones sesquiterpéniques comprenant de l'α-méthylène-γ-butyrolactone (α-MGBL), dans laquelle l'extrait de chicorée est obtenu par dégraissage d'une matière végétale de chicorée pour former un premier extrait végétal et ensuite en traitant le premier extrait végétal en utilisant une hydrolyse acide et une extraction avec un solvant au moyen d'acétate d'éthyle pour former l'extrait de chicorée.

2. Composition suivant la revendication 1, comprenant une quantité efficace d'un ou plusieurs agents phytochimiques qui comprennent une lactone sesquiterpénique comprenant un ingrédient actif de l'α-méthylène-γ-butyrolactone.

3. Composition suivant l'une quelconque des revendications 1 et 2, qui comprend 0,5 % d'extrait de chicorée extrudé en poids.

4. Composition suivant l'une quelconque des revendications précédentes, qui comprend en outre un agent diététique choisi dans le groupe consistant en des antioxydants, la glucosamine, le sulfate de chondroïtine, des acides gras oméga-3 et leurs associations.

5. Produit alimentaire qui est un aliment pour animaux de compagnie comprenant :
une matrice amylacée ; et
une composition suivant l'une quelconque des revendications 1 à 4.

6. Produit alimentaire suivant la revendication 5, choisi dans le groupe consistant en un supplément nutritionnel, un produit alimentaire nutritionnellement complet, une préparation alimentaire, un produit à base de céréales, un aliment pour animaux de compagnie et une préparation alimentaire pharmaceutique et/ou fonctionnelle.

7. Procédé pour la préparation d'un produit alimentaire nutritionnel pour la prévention ou le traitement de l'inflammation chez un mammifère, procédé comprenant les étapes consistant à :
fournir un extrait de chicorée extrudé obtenu en dégraissant une matière végétale de chicorée pour former un premier extrait végétal et ensuite en traitant le premier extrait végétal en utilisant une hydrolyse acide et une extraction avec un solvant au moyen d'acétate d'éthyle pour former l'extrait de chicorée, et à traiter l'extrait de chicorée et un ou plusieurs ingrédients alimentaires pour former un produit alimentaire nutritionnel qui comprend au moins 0,5 % en poids de l'extrait de chicorée.

8. Procédé suivant la revendication 7, dans lequel l'extrait de chicorée comprend un ou plusieurs agents phytochimiques capables d'inhiber l'activité enzymatique de la cyclooxygénase et/ou du facteur de transcription NF-κB.

9. Procédé suivant la revendication 7 ou 8, dans lequel l'extrait de chicorée extrudé comprend en outre un agent diététique choisi dans le groupe consistant en des antioxydants, la glucosamine, des acides gras oméga-3 et leurs associations.

10. Composition suivant l'une quelconque des revendications 1 à 4, destinée à être utilisée en thérapie.

11. Utilisation d'une composition suivant l'une quelconque des revendications 1 à 4 dans la production d'un médicament destiné à être utilisé dans le traitement de l'inflammation ou du cancer, ou la réduction du risque d'inflammation, d'arthrose, d'une maladie auto-immune ou du cancer, chez un mammifère.

12. Utilisation suivant la revendication 11, dans laquelle le médicament comprend 0,5 % en poids d'une composition suivant l'une quelconque des revendications 1 à 4.
